# EUROPEAN PATENT APPLICATION

(11) **EP 4 588 929 A1**
(43) Date of publication of application: **23.07.2025**
(21) Application number: 23865378.6
(22) Date of filing: 06.09.2023
(51) Int. Cl.: C07H 17/065, C07B 61/00

(54) **METHOD FOR PRODUCING PROTECTED GLYCOSIDE DERIVATIVE**

(30) Priority: 16.09.2022 JP 2022148337
(71) Applicant: Resonac Corporation, Tokyo 105-7325 (JP)
(72) Inventor: ISHIBASHI, Yoshitaka, Tokyo 105-7325 (JP); MIYATA, Toko, Tokyo 105-7325 (JP)
(74) Representative: Strehl & Partner mbB
(86) International application number: PCT/JP2023/032505
(87) International publication number: WO 2024/058023

(57) **Abstract**

Provided is a method for producing a protected glycoside derivative, including reacting a sugar in which all hydroxyl groups of the sugar are protected by protective groups and protective groups of a hydroxyl group at a 1-position and a hydroxyl group at a 2-position are acyl groups, with a phenolic compound in the presence of at least one catalyst selected from the group consisting of trifluoromethanesulfonic acid and copper (II) trifluoromethanesulfonate, in which the phenolic compound is bonded to an anomeric position of the sugar and other hydroxyl groups of the sugar are protected.

## Description

### TECHNICAL FIELD

The present invention relates to a method for producing a protected glycoside derivative in which a phenolic compound is bonded to an anomeric position of a sugar and other hydroxyl groups of the sugar are protected.

Priority is claimed on Japanese Patent Application No. 2022-148337, filed September 16, 2022, the content of which is incorporated herein by reference.

### BACKGROUND ART

One of compounds synthesized as a glycoside is tocopheryl glucoside, in which glucose is bonded to vitamin E. Tocopheryl glucoside is used as a derivative of tocopherol having various pharmacological activities such as peripheral vasodilator and histamine release inhibitory actions.

Glycoside is a general term for a compound in which an alcohol or a phenolic compound is bonded only to an anomeric position (1-position of a pyranose ring or a furanose ring) of a cyclic sugar via an ether bond. All the other hydroxyl groups of the cyclic sugar are not protected. The glycoside derivative is a compound in which any of the hydroxyl groups on a sugar skeleton are modified. In the present specification, "protected glycoside derivative" refers to a compound in which a hydroxyl group on a sugar skeleton of the glycoside or the glycoside derivative is protected.

Examples of the glycoside include a glucoside having a glucose skeleton, a fructoside having a fructose skeleton, a mannoside having a mannose skeleton, and a galactoside having a galactose skeleton.

Patent Documents 1 and 2 disclose examples of a method for producing a tocopheryl glucoside obtained by reacting a peracetylated sugar with vitamin E to protect a hydroxyl group on a sugar skeleton and deprotecting the protected hydroxyl group. Patent Document 1 describes a method for producing a glycoside derivative in which a hydroxyl group on a sugar skeleton is protected using an acid catalyst such as ferric chloride, cupric chloride, zinc chloride, aluminum chloride, boron trifluoride, titanium tetrachloride, or tin tetrachloride. In addition, Patent Document 2 describes a method for producing a glycoside derivative in which a hydroxyl group on a sugar skeleton is protected using an acid catalyst such as p-toluenesulfonic acid.

### Citation List

### Patent Documents

Patent Document 1: Japanese Unexamined Patent Application, First Publication No. H2-144151
Patent Document 2: Japanese Examined Patent Application, Second Publication No. H1-55278

### SUMMARY OF INVENTION

### Technical Problem

In the methods described in Patent Documents 1 and 2, the yield of the glycoside derivative in which the hydroxyl group of the sugar is protected in the reaction between the sugar in which the hydroxyl group is protected and the phenolic compound is not necessarily high. Therefore, there is a problem in that the yield of the deprotected glycoside derivative obtained after the subsequent deprotection reaction is also low.

Therefore, an object of the present invention is to provide a method for producing a protected glycoside derivative in which a glycoside derivative in which a hydroxyl group of a sugar is protected can be produced with a high yield.

### Solution to Problem

The present invention includes the following aspects.
[1] A method for producing a protected glycoside derivative, including: reacting a sugar in which all hydroxyl groups of the sugar are protected by protective groups and protective groups of a hydroxyl group at a 1-position and a hydroxyl group at a 2-position are acyl groups, with a phenolic compound in a presence of at least one catalyst selected from the group consisting of trifluoromethanesulfonic acid and copper (11) trifluoromethanesulfonate, in which the phenolic compound is bonded to an anomeric position of the sugar and other hydroxyl groups of the sugar are protected.
[2] The method for producing a protected glycoside derivative according to [1], in which the acyl group is an acetyl group.
[3] The method for producing a protected glycoside derivative according to [1] or [2], in which the sugar in which all the hydroxyl groups of the sugar are protected by the protective groups and the protective groups of the hydroxyl group at the 1-position and the hydroxyl group at the 2-position are acyl groups is a peracetylated sugar.
[4] The method for producing a protected glycoside derivative according to any one of [1] to [3], in which the sugar is glucose, and the phenolic compound is vitamin E.
[5] The method for producing a protected glycoside derivative according to any one of [1] to [4], in which 1 to 10 mol of the catalyst is used with respect to 100 mol of the phenolic compound in the reaction.

### Advantageous Effects of Invention

According to the present invention, it is possible to provide a method for producing a protected glycoside derivative in which a glycoside derivative, in which a hydroxyl group of a sugar is protected, can be produced with a high yield.

### DESCRIPTION OF EMBODIMENTS

Hereinafter, embodiments according to the present invention will be described in detail. However, the present invention is not limited to the embodiments described below.

### (Method for producing glycoside derivative)

In one embodiment, the present invention provides a method for producing a protected glycoside derivative in which a phenolic compound is bonded to an anomeric position of a sugar (which has a ring structure) and other hydroxyl groups of the sugar are protected. The production method of the present embodiment includes reacting a sugar in which all hydroxyl groups of the sugar are protected by protective groups and protective groups of a hydroxyl group at the 1-position and a hydroxyl group at the 2-position are acyl groups, with a phenolic compound in the presence of at least one catalyst selected from the group consisting of trifluoromethanesulfonic acid and copper (11) trifluoromethanesulfonate (hereinafter, also referred to as "step A").

### [Step A]

In the step A, a sugar in which all hydroxyl groups of the sugar are protected by protective groups and protective groups of a hydroxyl group at the 1-position and a hydroxyl group at the 2-position are acyl groups (hereinafter, also referred to as "protected sugar") reacts with a phenolic compound in the presence of at least one catalyst selected from the group consisting of trifluoromethanesulfonic acid and copper (II) trifluoromethanesulfonate.

### <Protected sugar>

The protective group in the sugar in which all hydroxyl groups of the sugar are protected by the protective group is preferably a protective group that does not cause a side reaction in the reaction with a phenolic compound. Examples of the protective group include an acyl type protective group such as an acetyl group or a benzoyl group; an ether type protective group such as a methyl group, an allyl group, or a benzyl group; and an acetal type protective group such as an isopropylidene group or a benzylidene group. The sugar can be converted into a protected sugar by a known method.

In the protected sugar used in the production method according to the present embodiment, the protective group of the hydroxyl group at the 1-position and the protective group of the hydroxyl group at the 2-position are acyl groups. Examples of the acyl type protective group include an acetyl group, a methoxyacetyl group, and a benzoyl group. Among these, an acetyl group is preferable.

As the protected sugar, a sugar in which all hydroxyl groups of the sugar are protected by an acyl group is preferable from the viewpoint that the synthesis thereof is easily carried out. Among these, a sugar in which all hydroxyl groups of the sugar are protected by an acetyl group is more preferable from the viewpoint of the availability. In the present specification, a sugar in which all hydroxyl groups of the sugar are protected by an acetyl group is also referred to as "peracetylated sugar".

The sugar in which the hydroxyl group is protected is not particularly limited. Examples of the sugar include hexoses such as glucose, mannose, galactose, fucose, and rhamnose; and pentoses such as ribose and arabinose. As the protected sugar, sugars in which all hydroxyl groups of these sugars are protected by an acyl group can be used.

As an example, a protected sugar (protected glucose) derived from β-glucose is shown in Formula (1). 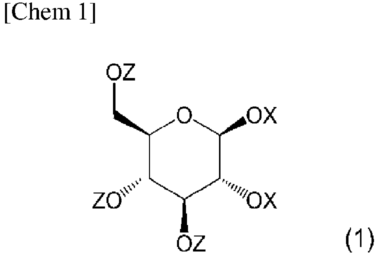 (In the formula, X's each independently represents an acyl group; and Z's each independently represents a protective group.)

In Formula (1), examples of the acyl group as X include the same groups as described above. Among these, an acetyl group is preferable. Examples of the protective group as Z include the same groups as described above. Among these, an acyl group is preferable, and an acetyl group is more preferable.

As the protected sugar, a peracetylated sugar of a hexose is preferable from the viewpoint of sufficient reactivity and availability. Examples of the hexose include glucose, mannose, galactose, fucose, and rhamnose.

The peracetylated sugar can be produced by acetylating a desired sugar by a known acetylation method. Examples of a known acetylation method include a method using acetic anhydride, sodium acetate, and acetyl chloride.

Specific examples of the peracetylated sugar include penta-O-acetyl-β-D-glucopyranose (pentaacetyl-β-D-glucose), penta-O-acetyl-α-D-glucopyranose (pentaacetyl-α-D-glucose), 1,2,3,4,6-penta-O-acetyl-D-mannopyranose, penta-O-acetyl-β-D-galactopyranose, and 1,2,3,4-tetra-O-acetyl-α-L-fucopyranose. As the peracetylated sugar, pentaacetyl-β-D-glucose or pentaacetyl-α-D-glucose is preferable, and penta-O-acetyl-β-D-glucose is more preferable.

### <Phenolic compound>

The term "phenolic compound" is a compound having a phenol skeleton. The phenol skeleton is a structure in which at least one hydrogen atom of a benzene ring is substituted with a hydroxyl group. The aromatic hydrocarbon ring in the phenol skeleton may form a fused ring with another aromatic ring or aliphatic ring. The phenolic compound is a compound selected from phenols. The phenolic compound is not particularly limited, and a known phenolic compound can be used. Examples of the phenolic compound include phenol, catechol, cresol, methoxyphenol, bromophenol, xylenol, ethylphenol, naphthol, resorcinol, hydroquinone, salicyl alcohol, and vitamin E. As the phenolic compound, vitamin E is preferable.

Vitamin E is tocopherol and tocotrienol. The tocopherol is a compound represented by Formula (2). The tocotrienol is a compound represented by Formula (3). 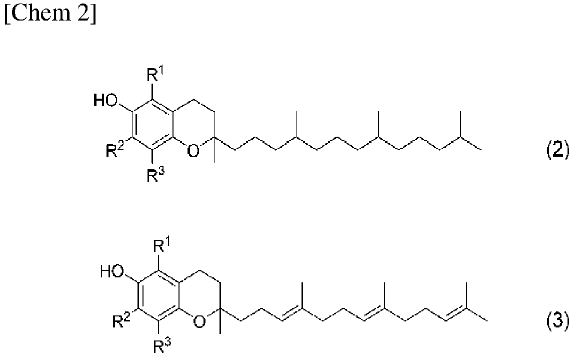 (In the formulae, R¹, R², and R³ each independently represents a hydrogen atom or a methyl group.)

The tocopherol and the tocotrienol are present as α-tocopherol and α-tocotrienol (R¹, R², and R³ = CH₃), β-tocopherol and β-tocotrienol (R¹ and R³ = CH₃, R² = H), γ-tocopherol and γ-tocotrienol (R¹ = H, R² and R³ = CH₃), and δ-tocopherol and δ-tocotrienol (R¹ and R² = CH₃, R³ = H) depending on R¹, R², and R³ in General Formulae (2) and (3).

In the case where vitamin E is used as the phenolic compound, any of α-tocopherol, β-tocopherol, γ-tocopherol, δ-tocopherol, α-tocotrienol, β-tocotrienol, γ-tocotrienol, or δ-tocotrienol may be used.

Since each of tocopherol and tocotrienol has an asymmetric carbon atom at the 2-position of the chroman ring, stereoisomers of a d form and an 1 form and a dl form are present. Each of the tocopherol and the tocotrienol may be any of these stereoisomers thereof.

### <Catalyst>

In the reaction between the protected sugar and the phenolic compound, at least one catalyst selected from the group consisting of trifluoromethanesulfonic acid and copper (11) trifluoromethanesulfonate is used as a catalyst. As the catalyst, any one of trifluoromethanesulfonic acid or copper (II) trifluoromethanesulfonate may be used alone, or a mixture of trifluoromethanesulfonic acid and copper (II) trifluoromethanesulfonate may be used.

In the reaction using trifluoromethanesulfonic acid and/or copper (II) trifluoromethanesulfonate as a catalyst, a hydroxyl group of a phenolic compound attacks the anomeric position of the protected sugar, and the acyl group that protects the hydroxyl group at the anomeric position is replaced with the phenolic compound. In this case, In the case where the hydroxyl group at the 2-position of the sugar is protected by an acyl group, a 1,2-trans-glycoside derivative is preferentially obtained by the involvement of adjacent groups.

### <Reaction>

The reaction between the protected sugar and the phenolic compound can be carried out by adding the reactant and the catalyst to a solvent. Examples of the usable solvent include toluene, benzene, ethylbenzene, xylene, ethyl acetate, methyl acetate, and methylcyclohexane. As the solvent, toluene, xylene, ethylbenzene, or benzene is preferable, and toluene is more preferable.

The reaction temperature and the reaction time can be suitably adjusted depending on the kind of the solvent and the catalyst used. The reaction temperature and the reaction time may be determined according to the progress of the reaction. The target reaction can usually be achieved by carrying out the reaction at about 0°C to 100°C for 0.5 to 24 hours under normal pressure. The reaction temperature may be, for example, 10°C to 80°C, 20°C to 70°C, 20°C to 60°C, 20°C to 50°C, or 20°C to 40°C. In the step A, the reaction can proceed under relatively mild temperature conditions by using trifluoromethanesulfonic acid or copper (II) trifluoromethanesulfonate as a catalyst.

The reaction time depends on the amount of the reactant, but may be, for example, 1 to 20 hours, 1 to 15 hours, 1 to 10 hours, 1 to 5 hours, or 2 to 5 hours.

The amount of the protected sugar to be used is preferably 1 to 1.5 molar equivalents with respect to the phenolic compound.

The total amount of the catalyst to be used is preferably 1 to 10 mol and more preferably 1 to 3 mol with respect to 100 mol of the phenolic compound.

A glycoside derivative (hereinafter, also referred to as "protected glycoside derivative") in which the phenolic compound is bonded to the anomeric position of the sugar and the hydroxyl group of the sugar is protected can be obtained by the reaction between the protected sugar and the phenolic compound. The glycosidic bond formed by the reaction between the sugar and the phenolic compound may be an α-glycosidic bond or a β-glycosidic bond. According to the method of the present embodiment, a glucoside derivative in which the glycosidic bond and the acyl group at the 2-position are in a trans relationship is obtained with satisfactory selectivity. Such a glucoside derivative is excellent from the viewpoint that the product is not a mixture with a glucoside derivative that is in a cis relationship and an isolation operation of the product, which is necessary in some cases, is not required. In the case where the phenolic compound is vitamin E, a bond between the sugar and -O- at the 6-position of the chroman ring of vitamin E is in a trans relationship with a hydroxyl group at the 2-position. The reason the glucoside derivative, which is in a trans relationship, is obtained with satisfactory selectivity is due to the effect of the involvement of adjacent groups of the acyl group, which is a protective group at the 2-position.

As an example, the reaction between the protected β-glucose and the phenolic compound is shown in Reaction Formula (1). A β-glucoside derivative in which the phenolic compound is bonded to the anomeric position of the β-glucose and the hydroxyl group of the β-glucose is protected can be obtained by the following reaction. 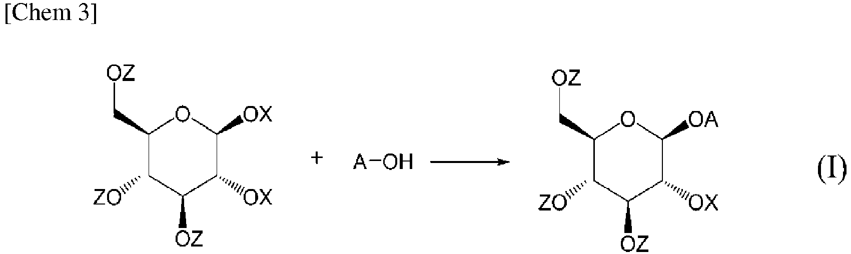 (In the formula, X's each independently represents an acyl group; and Z's each independently represents a protective group. A-OH represents a phenolic compound, and A represents an atomic group other than a hydroxyl group.)

In the step A, trifluoromethanesulfonic acid or copper (II) trifluoromethanesulfonate is used as a catalyst in the reaction of the sugar in which the hydroxyl group of the sugar is protected and the phenolic compound. A protected glycoside derivative can thus be obtained with a high yield of 70% or more.

After the step A, a glycoside in which the phenolic compound is bonded to the anomeric position of the sugar may be obtained by performing a deprotection reaction of the hydroxyl group of the sugar by the step B described below. Glycosides have attracted attention as pharmaceuticals, agricultural chemicals, cosmetics, reagents, and the like. The protected glycoside derivative obtained by the production method according to the present embodiment is useful as an intermediate of these useful glucosides.

### [Optional steps]

The method of the present embodiment may have optional steps in addition to the step A. Examples of the optional step include a step of deprotecting the protected hydroxyl group of the protected glycoside derivative obtained in the step A to obtain a glucoside (hereinafter, also referred to as "step B").

### <Step B>

After the step A, the protected hydroxyl group of the protected glycoside derivative obtained in the step A may be deprotected. The reaction conditions for deprotection can be suitably selected depending on the kind of the protective group. In the case where the protective group is an acyl group, the deprotection reaction can be carried out under basic conditions. As a base used for providing basic conditions, for example, a metal hydroxide such as sodium hydroxide or potassium hydroxide; or a metal alcoholate (metal alkoxide) such as sodium methoxide or sodium ethoxide can be used. As the solvent used in the deacylation reaction, water or an alcohol can be used in a case where a metal hydroxide is used as the base, and an alcohol can be used in a case where a metal alkoxide is used as the base. Examples of the alcohol include methanol.

The reaction conditions for the deprotection reaction can be suitably selected depending on the kind of the protective group. The reaction can usually be carried out at a temperature of about 0°C to 100°C for 0.5 to 24 hours under normal pressure. The reaction temperature may be, for example, 10°C to 80°C, 20°C to 70°C, 20°C to 60°C, 20°C to 50°C, or 20°C to 40°C. The reaction time depends on the amount of the reactant, but may be, for example, 0.5 to 20 hours, 0.5 to 15 hours, 0.5 to 10 hours, 0.5 to 5 hours, or 1 to 3 hours.

A glycoside in which the phenolic compound is bonded to the anomeric position of the sugar can be obtained by the step B.

As an example, a deprotection reaction of the protected β-glucoside derivative obtained by Reaction Formula (I) is shown in Reaction Formula (II). 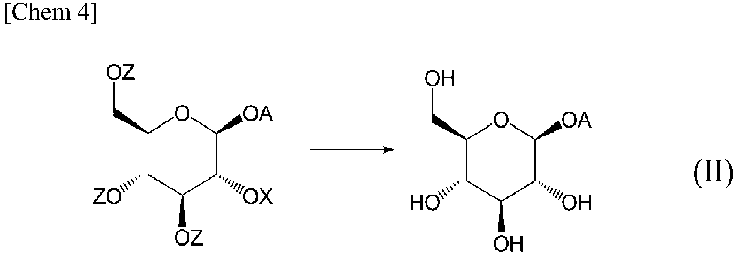 (In the formulae, X, Z, and A each has the same definition as described above.)

The method of the present embodiment may include a step of purifying the protected glycoside derivative obtained by the step A after the step A. **In** the case where the method of the present embodiment includes the step B, a step of purifying the glycoside obtained by the step B may be provided after the step B.

The purification can be performed by suitably combining known purification methods such as washing, drying under reduced pressure, solvent extraction, and chromatography.

In the case of including the step B, the production method of the present embodiment may be a method for producing a glycoside in which a phenolic compound is bonded to the anomeric position of the sugar.

According to the production method of the present embodiment, a glycoside derivative in which a hydroxyl group of a sugar is protected can be produced with high efficiency and a high yield in the step A, and thus a glycoside obtained by the deprotection reaction in the step B can also be obtained with high efficiency. Examples

Hereinafter, the present invention will be described in greater detail with examples and comparative examples, but is not limited to the following examples.

### (Example 1)

Pentaacetyl-β-D-glucose (Tokyo Chemical Industry Co., Ltd., 35.9 mmol, 14 g), d-δ-tocopherol (Sigma-Aldrich Co., LLC, 29.8 mmol, 12 g), trifluoromethanesulfonic acid (hereinafter, also referred to as "TfOH") (Kanto Chemical Co., Inc., 0.6 mmol, 0.09 g), and toluene (Kanto Chemical Co., Inc., 45 ml) were placed in a one-neck flask and stirred in an external bath at 30°C for 3 hours under normal pressure. Thereafter, the toluene layer after the reaction was subjected to liquid separation washing with 30 g of a NaHCO₃ saturated aqueous solution once and 30 g of distilled water once. In the case where the toluene layer was concentrated under reduced pressure, a mixture containing a glycoside derivative in which d-δ-tocopherol was bonded to the anomeric position and a hydroxyl group of a sugar was acetylated was obtained (24.5 g). The amount of target substance was 19.7 g (26.8 mmol, yield based on d-δ-tocopherol: 89.9%). 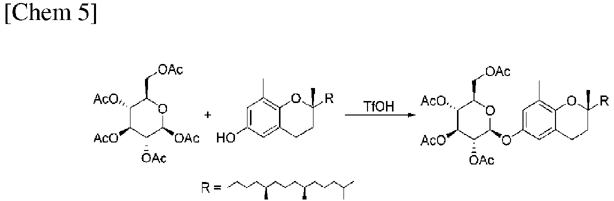 (In the formula, Ac represents an acetyl group.)

### (Reference Example: Synthesis of d-δ-tocopheryl β-d-glucopyranoside)

19.7 g (26.8 mmol) of the glycoside derivative in which d-δ-tocopherol obtained in Example 1 was bonded to the anomeric position and a hydroxyl group of a sugar was acetylated was heated and dissolved in methanol (150 ml, manufactured by FUJIFILM Wako Pure Chemical Corporation), and the solution was placed in a one-neck flask together with a 28% sodium methoxide-methanol solution (0.8 ml, manufactured by Seiko Pure Chemical Corporation), and a deacetylation reaction was carried out in an external bath at 30°C for 1 hour. After completion of the reaction, the filtrate was concentrated under reduced pressure, thereby obtaining 15 g (26.8 mmol, yield: 100%) of d-δ-tocopheryl β-d-glucopyranoside as a deacetylated product.

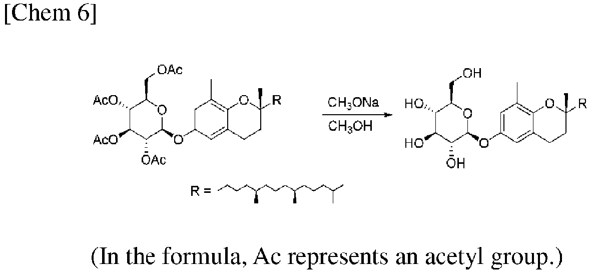

### (Example 2)

Pentaacetyl-β-D-glucose (Tokyo Chemical Industry Co., Ltd., 1.2 mmol, 0.468 g), d-δ-tocopherol (Sigma-Aldrich Co. LLC, 1.0 mmol, 0.403 g), copper (11) trifluoromethanesulfonate (Tokyo Chemical Industry Co., Ltd., 0.02 mmol, 0.007 g), and toluene (Kanto Chemical Co., Inc., 1.5 ml) were placed in a one-neck flask and stirred in an external bath at 30°C for 3 hours under normal pressure. Thereafter, the toluene layer after the reaction was subjected to liquid separation washing with 1 g of a NaHCO₃ saturated aqueous solution once and 1 g of distilled water once. In the case where the toluene layer was concentrated under reduced pressure, 0.8 g of a mixture containing a glycoside derivative in which d-δ-tocopherol was bonded to the anomeric position and hydrogen of another hydroxyl group was acetylated was obtained. The amount of target substance was 0.7 g (0.9 mmol, yield based on d-δ-tocopherol: 90.0%).

### (Comparative Example 1)

Pentaacetyl-β-D-glucose (Tokyo Chemical Industry Co., Ltd., 24.8 mmol, 10 g), d-δ-tocopherol (Sigma-Aldrich Co., LLC, 24.8 mmol, 10 g), a 4-toluenesulfonic acid monohydrate (Seiko Pure Chemical Industries, Ltd., 1.24 mmol, 0.236 g), and toluene (Kanto Chemical Co., Inc., 15 ml) were placed in a one-neck flask and stirred in an external bath at 70°C for 24 hours under normal pressure. Thereafter, the toluene layer after the reaction was subjected to liquid separation washing with 25 g of a NaHCO₃ saturated aqueous solution three times and 25 g of distilled water once. In the case where the toluene layer was concentrated under reduced pressure, 18.8 g of a mixture containing a glycoside derivative in which d-δ-tocopherol was bonded to the anomeric position and hydrogen of another hydroxyl group was acetylated was obtained. The target substance was 10.9 g (14.9 mmol, yield based on d-δ-tocopherol: 60%).

### (Comparative Example 2)

Pentaacetyl-β-D-glucose (Tokyo Chemical Industry Co., Ltd., 1.2 mmol, 0.468 g), d-δ-tocopherol (Sigma-Aldrich Co., LLC, 1.0 mmol, 0.403 g), zinc (II) trifluoromethanesulfonate (Tokyo Chemical Industry Co., Ltd., 0.02 mmol, 0.007 g), and toluene (Kanto Chemical Co., Inc., 1.5 ml) were placed in a one-neck flask and stirred in an external bath at 30°C for 3 hours under normal pressure, but the reaction did not proceed, and the raw materials were recovered (yield: 0%).

While preferred examples of the present invention have been described and illustrated above, it should be understood that these are examples of the present invention and are not to be considered as limiting. Additions, omissions, substitutions, and other modifications can be made without departing from the scope of the present invention. Accordingly, the present invention is not limited by the description above but by the scope of the appended claims.

## Claims

1. A method for producing a protected glycoside derivative, comprising:
reacting a sugar in which all hydroxyl groups of the sugar are protected by protective groups and protective groups of a hydroxyl group at a 1-position and a hydroxyl group at a 2-position are acyl groups, with a phenolic compound in a presence of at least one catalyst selected from the group consisting of trifluoromethanesulfonic acid and copper (II) trifluoromethanesulfonate,
wherein the phenolic compound is bonded to an anomeric position of the sugar and other hydroxyl groups of the sugar are protected.

2. The method for producing a protected glycoside derivative according to Claim 1,
wherein the acyl group is an acetyl group.

3. The method for producing a protected glycoside derivative according to Claim 1,
wherein the sugar in which all the hydroxyl groups of the sugar are protected by the protective groups and the protective groups of the hydroxyl group at the 1-position and the hydroxyl group at the 2-position are acyl groups is a peracetylated sugar.

4. The method for producing a protected glycoside derivative according to Claim 1 or 3,
wherein the sugar is glucose in which all the hydroxyl groups are protected by the protective groups and the protective groups of the hydroxyl group at the 1-position and the hydroxyl group at the 2-position are acyl groups, and the phenolic compound is vitamin E.

5. The method for producing a protected glycoside derivative according to Claim 1 or 3,
wherein 1 to 10 mol of the catalyst is used with respect to 100 mol of the phenolic compound in the reaction.
